# EUROPÄISCHE PATENTSCHRIFT

(11) **EP 1 244 674 B1**
(45) Veröffentlichungstag und Bekanntmachung des Hinweises auf die Patenterteilung: **17.03.2004**
(21) Anmeldenummer: 00991558.8
(22) Anmeldetag: 22.12.2000
(51) Int. Cl.: C07D 487/22, A01N 43/90

(54) **MIKROBIZIDE WIRKUNG VON PORPHYRINEN IM DUNKELN**
MICROBICIDAL EFFECT OF PORPHYRINES IN DARKNESS
ACTION MICROBICIDE DE PORPHYRINES A L'OBSCURITE

(30) Priorität: 23.12.1999 DE 19962505
(43) Veröffentlichungstag der Anmeldung: 02.10.2002
(73) Patentinhaber: Schaffer, Moshe, 81377 Munich (DE); Jori, Giulio, 35100 Padova (IT); Holtz, Alexander, 82031 Grünwald (DE)
(72) Erfinder: Schaffer, Moshe, 81377 Munich (DE); Jori, Giulio, 35100 Padova (IT); Holtz, Alexander, 82031 Grünwald (DE)
(74) Vertreter: VOSSIUS & PARTNER
(86) Internationale Anmeldenummer: PCT/DE2000/004609
(87) Internationale Veröffentlichungsnummer: WO 2001/047932

(56) Entgegenhaltungen:
- WO-A-93/00815
- DATABASE CHEMABS [Online] CHEMICAL ABSTRACTS SERVICE, COLUMBUS, OHIO, US; MADAKYAN, V. N. ET AL: "N,N',N",N'"-Tetrakis(1-alkylpyridinium-4- yl)mesoporphines and their biological activity" retrieved from STN Database accession no. 105:172135 XP002174170 & ARM. KHIM. ZH. (1985), 38(6), 391-6 ,
- DATABASE CHEMABS [Online] CHEMICAL ABSTRACTS SERVICE, COLUMBUS, OHIO, US; FOURNIER, T. ET AL: "Optical properties of transient charge carriers photogenerated on a femtosecond-to-nanosecond time scale in Langmuir-Blodgett films" retrieved from STN Database accession no. 122:146844 XP002174171 & THIN SOLID FILMS (1994), 242(1-2), 92-5 ,
- DATABASE CHEMABS [Online] CHEMICAL ABSTRACTS SERVICE, COLUMBUS, OHIO, US; HUEGLIN, D. ET AL: "Time-resolved microfluorometric study of the binding sites of lipophilic cationic pyrene probes in mitochondria of living HeLa cells" retrieved from STN Database accession no. 124:140109 XP002174172 & J. PHOTOCHEM. PHOTOBIOL., B (1995), 31(3), 145-58 ,
- DATABASE CHEMABS [Online] CHEMICAL ABSTRACTS SERVICE, COLUMBUS, OHIO, US; OKUNO, YOHMEI ET AL: "An improved synthesis of surfactant porphyrins" retrieved from STN Database accession no. 93:106192 XP002174173 & SYNTHESIS (1980), (7), 537-9 ,
- DATABASE CHEMABS [Online] CHEMICAL ABSTRACTS SERVICE, COLUMBUS, OHIO, US; NAGAO, TAKUMI: "Optical recording media containing porphyrin compound" retrieved from STN Database accession no. 131:191925 XP002174174 & JP 11 221964 A (MITSUBISHI CHEMICAL INDUSTRIES LTD., JAPAN) 17. August 1999 (1999-08-17)
- DATABASE CHEMABS [Online] CHEMICAL ABSTRACTS SERVICE, COLUMBUS, OHIO, US; MADAKYAN, V. N. ET AL: "New N,N',N",N'"-tetrakis(1-alkylpyridinium-4-y l)mesoporphines and their metal complexes" retrieved from STN Database accession no. 105:172134 XP002174175 & ARM. KHIM. ZH. (1985), 38(6), 386-91 ,

## Beschreibung

Die vorliegende Erfindung betrifft Porphyrinderivate, welche eine mikrobizide Wirkung aufweisen.

Porphyrine sind Photosensibilisatoren, welche elektromagnetische Strahlung, vorzugsweise sichtbares Licht, absorbieren und dadurch die Bildung von Radikalen und/oder Singulett-Sauerstoff aus Triplett-Sauerstoff katalysieren. Durch diesen Radikalmechanismus werden lebende Zellen teilweise oder ganz zerstört, und somit wird eine Möglichkeit zur Bekämpfung von schädlichen Lebewesen eröffnet. So offenbart z.B. EP-A-0 390 743 Porphyrine und Phthalocyanine als Insektizide und Akarizide. US-A-4,648,992 offenbart wasserlösliche Phthalocyaninverbindungen, die als Bleichmittel oder auch zur Desinfektion von Schwimmbädern Verwendung finden können. DE-A-196 06 082 betrifft ein Schädlingsbekämpfungsmittel, das einen Photosensibilisator der Tetrapyrrol- und/oder Tetraazapyrrolreihe enthält. In DE-A-196 06 081 werden ähnliche Tetrapyrrol-Photosensibilisatoren zum Bekämpfen von Bakterien in Wasser eingesetzt.

Allen vorstehenden Anwendungen ist jedoch gemeinsam, daß die Photosensibilisatoren nur unter Einwirkung von elektromagnetischer Strahlung/Licht wirksam sind.

Es wurde nun überraschend gefunden, daß bestimmte Porphyrinderivate auch ohne Einfluß von elektromagnetischer Strahlung eine mikrobizide Wirkung zeigen.

Erfindungsgemäß werden somit Verbindungen der folgenden allgemeinen Formel (I) bereitgestellt: 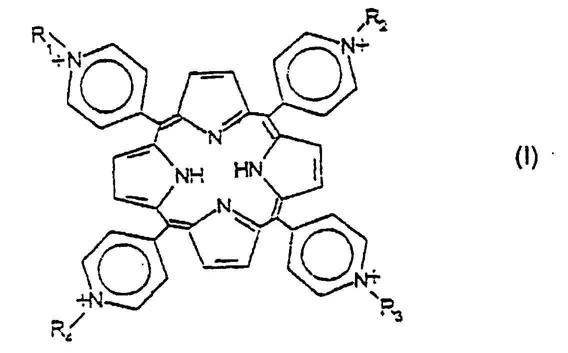 wobei
- R₁=R₂=R₃=-CH₃ und R₄=: ―CH₂(CH₂)₄CH₃ mono-C6
―CH₂(CH₂)₈CH₃ mono-C 10
―CH₂(CH₂)₁₂CH₃ mono-C14
―CH₂(CH₂)₁₆CH₃ mono-C 18
―CH₂(CH₂)₂₀CH₃ mono-C22 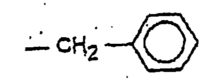 mono-benzyl
bedeuten. beanspruchten Verbindungen können als Mikrobizide, insbesondere Bakterizide, eingesetzt werden.

Die erfindungsgemäßen Verbindungen können in analoger Weise zu bereits bekannten Porphyrinderivaten hergestellt werden.

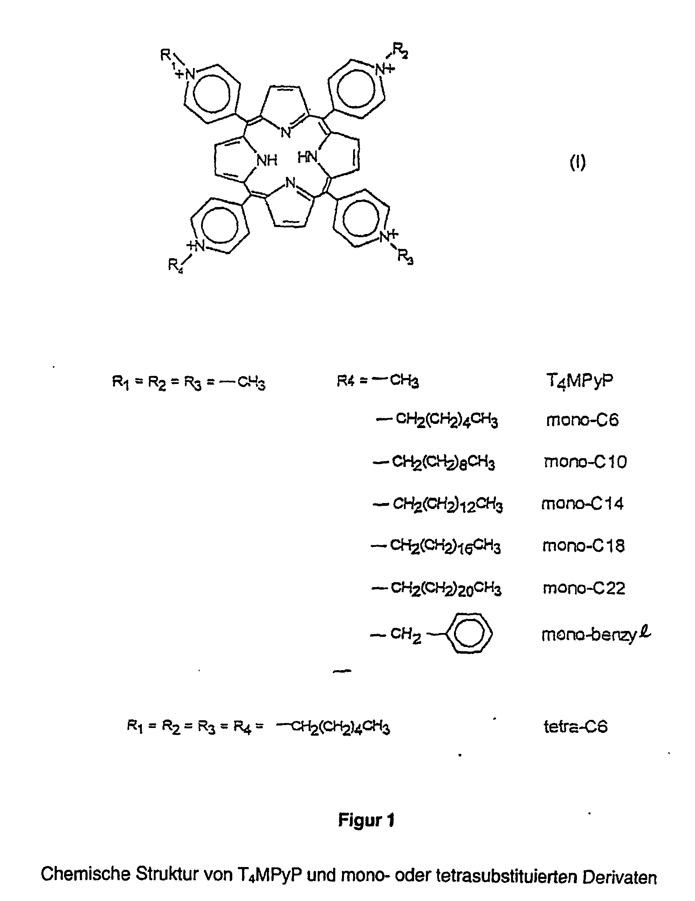

Die Wirksamkeit der erfindungsgemäßen Verbindungen wird in den nachstehenden Beispielen illustriert. Die Definitionen der verwendeten Porphyrinderivate sind aus Fig. 1 zu entnehmen.

Dazu wurden Porphyrine unterschiedlicher chemischer Struktur (siehe Zeichnung) im Dunkeln mit *E. coli* und *S*. *aureus* Zellen inkubiert. Die Porphyrine wurden wäßrigen Suspensionen mit einem Gehalt an bakteriellen Zellen von 10⁸ pro ml zugegeben und bei Raumtemperatur 5 Minuten im Dunkeln gelagert. Schließlich wurde die Menge des an die Zellen gebundenen Porphyrins in Abhängigkeit der Anzahl an Waschschritten (das Waschen wurde mittels physiologischer Lösungen durchgeführt) durch chemische Extraktion gefolgt von spektrophotofluorimetrischer Bestimmung gemessen.

Die Überlebensrate der bakteriellen Zellen nach der Inkubation mit den verschiedenen Porphyrinen wurde bestimmt durch Verdünnen von 0,3 ml der Suspension mit 2,7 ml des Nährmediums und Aufzeichnen der optische Dichte bei 650 nm in Abhängigkeit von der Zeit. Die Suspensionen wurden bei 37°C gehalten. Die prozentuale Wachstumshemmung wurde mit Bezug auf eine Suspension von Vergleichszellen berechnet, die mit dem gleichen Verfahren behandelt, jedoch nicht dem Porphyrin zugesetzt worden war.

**Tabelle 1**

| Gewinnung von Porphyrin aus *E. coli* Zellen, die 5 Minuten im Dunkeln mit 1 µM Porphyrin inkubiert und verschiedenen Waschschritten unterzogen wurden | | | | |
|---|---|---|---|---|
| Porphyrin | Gewinnung (nmol/mg Protein) | | | % gebunden nach 3 x Waschen |
| | kein Waschen | 1 x Waschen | 3x Waschen | |
| T₄MPyP | 0,44 ± 0,04 | 0,19 ± 0,04 | 0,05 ± 0,01 | 11,4 |
| mono-C6 | 1,58 ± 0,28 | 1,01 ± 0,12 | 0,39 ± 0,12 | 24,7 |
| mono-C10 | 3,66 ± 0,071 | 2,78 ± 0,53 | 1,48 ± 0,38 | 40,4 |
| mono-C14 | 5,38 ± 0,06 | 3,06 ± 0,25 | 2,28 ± 0,04 | 42,4 |
| mono-C18 | 4,20 ± 0,16 | 4,08 ± 0,62 | 2,54 ± 0,38 | 60,5 |
| mono-C22 | 5,12 ± 0,46 | 2,60 ± 0,04 | 3,04 ± 0,34 | 59,4 |
| mono-Benzyl | 2,52 ± 0,13 | 1,67 ± 0,06 | 0,71 ± 0,08 | 28,2 |
| tetra-C6 | 1,65 ± 0,17 | 1,39 ± 0,23 | 0,94 ± 0,24 | 57,0 |

**Tabelle 2**

| Gewinnung von Porphyrin aus *S. aureus* Zellen, die 5 Minuten im Dunkeln mit 1 µM Porphyrin inkubiert und verschiedenen Waschschritten unterzogen wurden | | | | |
|---|---|---|---|---|
| Porphyrin | Gewinnung (nmol/mg Protein) | | | % gebunden nach 3 x Waschen |
| | kein Waschen | 1 x Waschen | 3x Waschen | |
| T₄MPyP | 0,68 ± 0,05 | 0,10 ± 0,01 | 0,11 ± 0,01 | 16,2 |
| mono-C6 | 3,04 ± 0,05 | 2,05 ± 0,11 | 1,02 ± 0,12 | 33,6 |
| mono-C10 | 7,13 ± 0,25 | 5,70 ± 0,54 | 4,09 ± 0,13 | 57,4 |
| mono-C14 | 6,56 ± 0,40 | 5,53 ± 0,74 | 4,32 ± 0,13 | 65,9 |
| mono-C22 | 6,39 ± 0,40 | 5,65 ± 0,13 | 4,95 ± 0,30 | 77,5 |

**Tabelle 3**

| Auswirkung der 5-minütigen Inkubation im Dunkeln von *E. coli* und *S. aureus* Zellen mit den verschiedenen Porphyrinen auf die Überlebensrate der bakteriellen Zellen | | | |
|---|---|---|---|
| Porphyrin | Konzentration (µM) | Wachtumshemmung (%) | |
| | | *E. coli* | *S. aureus* |
| T₄MPyP | 1 | 0,6 | 3,1 |
| | 10 | 3,1 | 7,8 |
| mono-C6 | 1 | 0,5 | 5,1 |
| | 10 | 14,6 | 8,8 |
| Mono-C10 | 1 | 4,6 | 27,8 |
| | 10 | 10,7 | 50 |
| Mono-C14 | 1 | 46,1 | 69,1 |
| | 10 | 100 | 92,4 |
| Mono-C18 | 1 | 2,7 | 39,8 |
| | 10 | 85,7 | 96,6 |
| Mono-C22 | 1 | 19,1 | 39,9 |
| | 10 | 16,8 | 70,1 |
| Mono-Benzyl | 1 | 0,1 | 1,6 |
| | 10 | 13 | 25 |

## Patentansprüche

1. Verbindung der Formel: 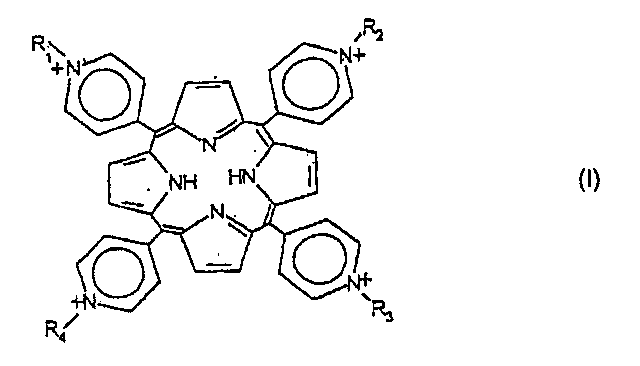 wobei
R₁=R₂=R₃= -CH₃ und R₄= ―CH₂(CH₂)₄CH₃ mono-C6
―CH₂(CH₂)₈CH₃ mono-C 10
―CH₂(CH₂)₁₂CH₃ mono-C14
―CH₂(CH₂)₁₆CH₃ mono-C 18
―CH₂(CH₂)₂₀CH₃ mono-C 22 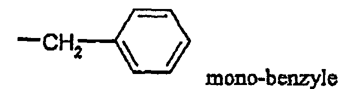
bedeuten.

2. Verwendung einer Verbindung nach Anspruch 1 als Mikrobizid, welches ohne Einfluß von elektromagnetischer Strahlung eine mikrobizide Wirkung zeigt.

3. Verwendung nach Anspruch 2, wobei die mikrobizide Wirkung im wesentlichen in Abwesenheit von sichtbarem und UV-Licht (im Dunkeln) erfolgt.

4. Verwendung nach Anspruch 3, wobei die mikrobizide Wirkung im Dunkeln und unter Einwirkung von sichtbarem und UV-Licht erfolgt.

5. Verwendung nach Anspruch 4, wobei die Wellenlänge von sichtbarem und UV-Licht im Bereich von 350 bis 900 nm liegt.

## Claims

1. A compound of the formula: 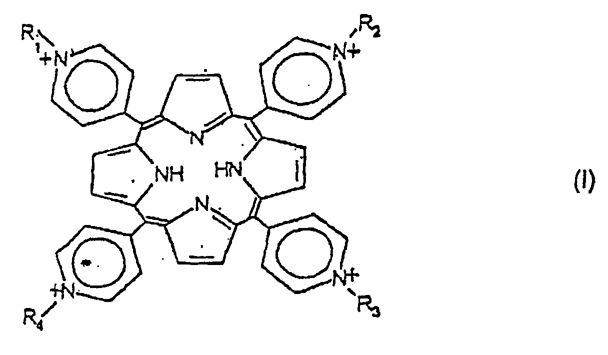 wherein
R₁ =R₂ =R₃ is CH3 and R₄ is ―CH₂(CH₂)₄CH₃ mono-C6
―CH₂(CH₂)₈CH₃ mono-C 10
―CH₂(CH₂)₁₂CH₃ mono-C14
―CH₂(CH₂)₁₆CH₃ mono-C 18
―CH₂(CH₂)₂₀CH₃ mono-C22

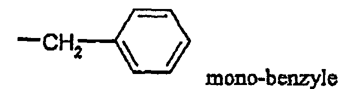

2. Use of the compound according to claim 1 as a microbicide which exhibits its microbicidal activity even in the absence of electromagnetic radiation.

3. Use according to claim 2, wherein the microbicial activity is exhibited essentially in the absence of visible and UV-light (in the dark).

4. Use according to claim 3, wherein the microbicidal activity is exhibited in the dark as well as under the influence of visible and UV-light.

5. Use according to claim 4, wherein the wave length of the visible and UV-light is in the range of 350-900 nm.

## Revendications

1. Composé de formule 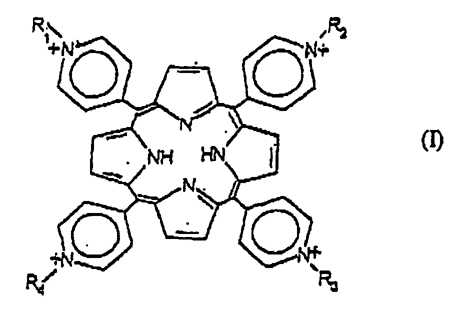 dans lequel
R₁ =R₂ = R₃ signifie -CH3 et R₄ signifie
- CH₂(CH₂)₄CH₃ mono-C6
-CH₂(CH₂)₈CH₃ mono-C10
-CH₂(CH₂)₁₂CH₃ mono-C14
- CH₂(CH₂)₁₆CH₃ mono-C18
- CH₂(CH₂)₂₀CH₃ mono-C22

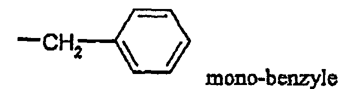

2. Utilisation d'un composé selon la revendication 1 comme microbicide, lequel montre un effet microbicide sans l'influence d'un rayonnement électromagnétique.

3. Utilisation selon la revendication 2, dans laquelle l'effet microbicide a lieu essentiellement en l'absence de lumière visible et UV (dans l'obscurité).

4. Utilisation selon la revendication 3, dans laquelle l'effet microbicide a lieu dans l'obscurité et sous l'influence de lumière visible et UV.

5. Utilisation selon la revendication 4, dans laquelle la longueur d'onde de lumièro visible et UV est comprise dans la gamme de 350 à 900 nm.
